# EUROPÄISCHE PATENTANMELDUNG

(11) **EP 4 785 998 A2**
(43) Veröffentlichungstag der Anmeldung: **05.08.2026**
(21) Anmeldenummer: 26186404.5
(22) Anmeldetag: 06.04.2023
(51) Int. Cl.: A61P 17/16

(54) **ZUSAMMENSETZUNG ZUR BEHANDLUNG DER HAUT**

(30) Priorität: 06.04.2022 DE 102022108329
(62) Teilanmeldung aus: 23719310.7
(71) Anmelder: Dr. Kurt Wolff GmbH & Co. KG, 33611 Bielefeld (DE)
(72) Erfinder: Dr. SCHULZE ZUR WIESCHE, Erik, 33611 Bielefeld (DE); Dr. BECKER, Maike, 33611 Bielefeld (DE); Dr. VÖLKER, Jörn Michael, 33611 Bielefeld (DE)
(74) Vertreter: Weickmann & Weickmann PartmbB

(57) **Zusammenfassung**

Die vorliegende Erfindung betrifft eine topische Zusammensetzung, die Dimethylglycin und/oder ein Salz von Dimethylglycin und mindestens eine Carbonsäure in einem Gewichtsverhältnis 1:0,1 bis 1:10 enthält zur Verwendung bei der Förderung von Wundheilung der Haut..

## Beschreibung

Die vorliegende Erfindung betrifft eine Zusammensetzung, die Dimethylglycin und/oder ein Salz von Dimethylglycin enthält zur Verwendung bei der Förderung von Wundheilung der Haut.

Die Haut ist das größte Organ des Menschen. Unter ihren vielen Funktionen (beispielsweise zur Wärmeregulation und als Sinnesorgan) ist die Barrierefunktion sowie Schutzfunktion, die das Austrocknen der Haut (und damit letztlich des gesamten Organismus) verhindert, die wohl Wichtigste. Gleichzeitig wirkt die Haut als Schutz gegen das Eindringen und die Aufnahme von externen Stoffen. Insbesondere sind hier schädliche UV-Strahlen zu nennen, mikrobiologische Angriffe und Penetration von hautfremden Schadstoffen. Bewirkt wird diese Barrierefunktion durch die Epidermis (Oberhaut), welche als äußerste Schicht die eigentliche Schutzhülle gegenüber der Umwelt bildet. Besondere Beachtung gilt der äußeren Hornschicht (Stratum Corneum), einer widerstandsfähigen Zellschicht, deren einzelne Hornzellen durch die sogenannte Lipid-Barriere verbunden sind. Die Lipid-Barriere schützt die Haut vor Austrocknung und hält den Quellungszustand aufrecht. Der natürliche pH-Wert der Hautoberfläche liegt in der Regel zwischen pH 4-7. Mit etwa einem Zehntel der Gesamtdicke der Haut ist die Epidermis gleichzeitig die dünnste Schicht der Haut. Diese umfasst, neben der Epidermis noch das darunter gelegene mesodermale Bindegewebe des Coriums (Lederhaut, Unterhaut). Beide Schichten sind durch ein System papillen- oder leistenförmiger Coriumsvorwölbungen und tief zwischen diese hineinragender Epidermiszapfen innig miteinander verzahnt. Vom reich durchbluteten Corium her folgt auch die Ernährung der Epidermis. Pigmentzellen im Corium liefern die Pigmente, die der Haut ihre Färbung verleihen und in den Coriumpapillen liegen die Sinnesrezeptoren der Hautsinne für Tastempfindung, Schmerzempfindung und Temperaturempfindung.

Die oberflächlichen Zelllagen der Epidermis unterliegen einem ständigen Verschleiß. Zusätzlich kann die Oberhaut sehr empfindlich mit Reizungen, Rötungen, Juckreiz, Schuppungen und Rissbildungen auf äußere Einflüsse reagieren. Zudem ist sie permanent der Gefahr von Entzündungen und Verletzungen (Wunden) ausgesetzt. Um daher den Schutz optimal zu gewährleisten, erneuert sich die Epidermis deshalb innerhalb von ca. 30 Tagen vollständig. Diese Epidermiserneuerung erfolgt aus einer basalen, zeitlebens teilungsaktiven Keimschicht durch eine kontinuierliche Ersetzung.

Kosmetische und dermatologische Zubereitungen für die Pflege der Haut sind bekannt. So basieren derartige Zusammensetzungen auf der Grundlage von O/W- oder W/O-Emulsionen, die beispielsweise auf einer Mischung aus langkettigen Fettsäuren, Mono- und/oder Diglyceriden von Fettsäuren, ethoxylierten Fettsäureestern, unpolaren Lipiden, Fettalkoholen, lipophilen Konsistenzgebern, hydrierten Polyisobutenen und polaren Lipiden basieren.

Insbesondere um die Hautbarriere der Epidermis zu verbessern, werden inzwischen viele kosmetische Zusammensetzungen angeboten, durch welche üblicherweise von außen Fettstoffe der Haut zugeführt werden. Solche Zusammensetzungen haben aber eine Reihe von Nachteilen. Die Penetration in die Haut ist oft eher gering, so dass das Hautgefühl unangenehm fettig sein kann. Meist sind die Fettstoffe auch körperfremd und können so eine geschwächte Hautbarriere nur unbefriedigend regenerieren. Mit zunehmendem Alter nimmt zusätzlich die Aktivität der Keratinozyten ab, d.h. die Zellteilung bzw. Hautregeneration und somit die Bildung der körpereigenen Hautbarriere nimmt ab.

Damit besteht ein Bedarf an verbesserten pharmazeutischen und kosmetischen Behandlungsmethoden, insbesondere ein Bedarf an Zusammensetzungen, die sowohl die Schutz- und Barrierefunktion der Haut stärken als auch gleichzeitig das Hautgefühl und das Hautbild verbessern, wobei diese Zusammensetzungen keine oder nur vernachlässigbare Nebenwirkungen zeigen sollen. Es besteht insbesondere ein Bedarf an Zusammensetzungen, die die Erneuerung der Epidermis unterstützen und Beeinträchtigungen der Haut durch Vorbelastungen, wie unter anderem schlecht heilende Wunden, Irritationen, Reizungen, Entzündungen und andere, ausgleichen.

Ausgehend hiervon war es die Aufgabe der vorliegenden Erfindung, eine gut verträgliche Zusammensetzung zur Behandlung der Haut, insbesondere zur Stärkung der Schutz- und Barrierefunktion der Haut, bereitzustellen. Weiterhin sollte sich diese Zusammensetzung topisch anwenden lassen und die Nachteile der aus dem Stand der Technik bekannten Zusammensetzungen überwinden.

Insbesondere war es die Aufgabe der vorliegenden Erfindung, eine wirksame und gut verträgliche Zusammensetzung vorzusehen, die unterstützend auf die Regeneration der Epidermis wirkt und die Wundheilung positiv beeinflusst. Gleichzeitig sollte die Zusammensetzung gut verarbeitbar sein.

Diese Aufgabe wurde überraschend durch die Zusammensetzung gemäß Anspruch 1 gelöst. Entsprechend betrifft die Erfindung eine topische Zusammensetzung umfassend
a) Dimethylglycin und/oder ein Salz von Dimethylglycin,
b) mindestens eine Carbonsäure,
wobei das Gewichtsverhältnis von a) zu b) von 1:0,1 bis 1:10, bevorzugt von 1:0,7 bis 1:1,5, reicht, zur Verwendung bei der Förderung von Wundheilung der Haut bzw. zur Verwendung zur Behandlung der Haut zur Förderung der Wundheilung.

. Bevorzugte Ausführungsformen gehen aus den abhängigen Ansprüchen hervor.

Erfindungsgemäß wird die Aufgabe durch die Bereitstellung einer topischen Zusammensetzung gelöst, welche Dimethylglycin und/oder ein Salz von Dimethylglycin und mindestens eine Carbonsäure in einem spezifischen Gewichtsverhältnis umfasst.

Es hat sich überraschend gezeigt, dass die erfindungsgemäße topische Zusammensetzung mit einer Kombination aus Dimethylglycin und/oder einem Salz von Dimethylglycin und mindestens einer Carbonsäure eine ausgezeichnete Wirksamkeit in der Stärkung der Schutz- und Barrierefunktion der Haut aufweist. Die Zusammensetzung aktiviert die Haut und verbessert die Nährstoff- und Sauerstoffversorgung der Haut und der Haarwurzel deutlich. Zudem ist sie auf der Haut medizinisch und kosmetisch äußerst verträglich. Ohne an eine Theorie gebunden zu sein, wird vermutet, dass die erfindungsgemäße topische Zusammensetzung überraschenderweise die Aktivität der Keratinozytenzellen steigert und so die Stärkung der Hautbarriere bewirkt bzw. positiv beeinflusst. Zudem wurde überraschend gefunden, dass die Zusammensetzung durch die kombinierte Anwesenheit von a) Dimethylglycin und/oder einem Salz von Dimethylglycin und b) einer Carbonsäure gemäß der Erfindung die Hautfeuchtigkeit steigert, den osmotischen Druck der Zusammensetzung (d.h. die Gesamtzahl der gelösten chemischen Einheiten in der Zusammensetzung) erhöht, die Freisetzung des Wirkstoffs (d.h. Dimethylglycin und/oder ein Salz von Dimethylglycin) aus der Zusammensetzung beschleunigt und insgesamt verbessert und die Penetration des Wirkstoffs in und durch die Haut beschleunigt und allgemein verbessert.

N,N-Dimethylglycin kommt natürlicherweise in Tieren und Pflanzen vor und ist ein Intermediat im Metabolismus von Cholin. N,N-Dimethylglycin ist per chemischer Definition der Säurekonstante eine schwache Säure und bildet in wässrigen Systemen eine alkalische Lösung. *N,N-*Dimethylglycin, auch (Dimethylamino)essigsäure, wird durch die folgende chemische Formel 1 dargestellt:

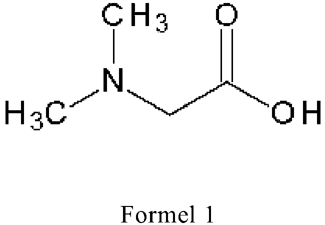

Erfindungsgemäß ist nicht nur der Einsatz von Dimethylglycin, sondern auch der von dessen Salzen, Solvaten und Hydraten. Dabei handelt es sich bevorzugt um pharmazeutisch bzw. kosmetisch annehmbare Salze von Dimethylglycin. Das Salz ist besonders bevorzugt ein wasserlösliches Salz mit einer Löslichkeit in Wasser von mindestens 10 g/l bei 20°C. Explizit nicht unter diese Formel sollen Betain-Strukturen und Betain-ähnlichen Strukturen fallen, d.h. Verbindungen mit vollständig alkylierten Ammoniumeinheiten (nämlich jegliche Verbindungen mit 4-fach C-substituiertem N-Atom).

In einer bevorzugten Aufführungsform ist das Salz von Dimethylglycin ein Alkali-, Erdalkali- oder Ammoniumsalz von Dimethylglycin.

Beispiele sind Natrium-, Kalium-, Calcium-, Magnesium- und Ammoniumsalze. Bei den Ammoniumsalzen trägt das Ammoniumkation eine bis vier Alkylgruppen mit jeweils unabhängig voneinander 1 bis 4 Kohlenstoffatomen. Bevorzugt sind das Natrium- und Kaliumsalz von Dimethylglycin, insbesondere das Natriumsalz von Dimethylglycin, nämlich Natrium-N,N-dimethylglycinat.

In einer alternativ bevorzugten Ausführungsform kann das Salz von Dimethylglycin das Salz einer anorganischen und/oder organischen Säure mit Dimethylglycin sein.

Beispiele für Salze von Dimethylglycin mit einer anorganischen Säure sind das Hydrochlorid, Hydrobromid, Hydroiodid, Hydrogensulfat, Sulfat, Hydrogensulfit, Sulfit, Hydrogencarbonat, Carbonat, Monophosphat, Diphosphat und Triphosphat von Dimethylglycin sowie Mischungen daraus. Insbesondere bevorzugt ist das Hydrochlorid von Dimethylglycin.

Beispiele für Salze von Dimethylglycin mit einer organischen Säure sind das Acetat, Laktat, Citrat, Succinat, Fumarat, Maleat und Benzoat von Dimethylglycin sowie Mischungen daraus.

Der Einsatz von Dimethylglycin und/oder einem Salz von Dimethylglycin in der erfindungsgemäßen topischen Zusammensetzung unterstützt die Regenerierung der Epidermis sowie die Wundheilung, glättet die Haut, stärkt die Schutz- und Barrierefunktion der Haut und hat einen positiven Effekt auf Haut mit entzündlichen Zuständen. Darüber hinaus wurde gefunden, dass auch die Aktivität der Keratinozyten durch Dimethylglycin und/oder ein Salz von Dimethylglycin deutlich gesteigert wurde. Damit dient es erfindungsgemäß sowohl zur Prophylaxe als auch zur Therapie von Störungen, die durch die mangelnde Regeneration der Epidermis hervorgerufen werden.

Es wird angenommen, dass Dimethylglycin und/oder ein Salz von Dimethylglycin erfindungsgemäß die Zellaktivität und den Sauerstoffumsatz in den Keratinozyten verbessert und damit auch die Zellaktivität in der Haut fördert. Es erzielt so erfindungsgemäß eine deutliche hautstärkende Wirkung, insbesondere bei der Behandlung von alltags- bzw. altersbedingt gestresster oder geschwächter Haut.

Neben Dimethylglycin und/oder Salzen von Dimethylglycin enthalten die erfindungsgemäßen Zusammensetzungen mindestens eine Carbonsäure. Bevorzugt ist die Carbonsäure ausgewählt aus der Gruppe bestehend aus Adipinsäure, Ascorbinsäure, Milchsäure, Essigsäure, Propansäure, Glykolsäure, Brenztraubensäure, Oxalsäure, Äpfelsäure, Malonsäure, Bernsteinsäure, Maleinsäure, Fumarsäure, Weinsäure, Zitronensäure, Benzoesäure, Zimtsäure, Mandelsäure, Methansulfonsäure, Ethansulfonsäure, p-Toluolsulfonsäure, Salicylsäure, Azelainsäure, Zimtsäure, Ameisensäure, (Meth)Acrylsäure, Nicotinsäure sowie Mischungen daraus. Ganz besonders bevorzugt dabei sind Milchsäure, Zitronensäure, Salicylsäure, Äpfelsäure, Fumarsäure und Weinsäure sowie Mischungen von zwei oder mehreren dieser Carbonsäuren.

Die mindestens eine Carbonsäure stellt dabei nicht auf die Haut abgestimmten pH-Wert der Zusammensetzung ein, sondern verstärkt in synergistischer Weise auch überraschend die oben genannten Effekte von Dimethylglycin und/oder den Salzen von Dimethylglycin auf die Haut. So resultiert eine Steigerung der Aktivität der Keratinozytenzellen und die Stärkung der Hautbarriere, die Erhöhung der Hautfeuchtigkeit und des osmotischen Drucks der Zusammensetzung, die Verbesserung und Beschleunigung der Freisetzung des Wirkstoffs aus der Zusammensetzung und eine Beschleunigung und Verbesserung der Penetration in und durch die Haut.

Das Gewichtsverhältnis von Dimethylglycin bzw. einem Salz von Dimethylglycin zu Carbonsäure beträgt 1:0,1 bis 1:10. Bevorzugt reicht das Verhältnis von 1:0,5 bis 1:5, besonders bevorzugt von 1:0,6 bis 1:2, am meisten bevorzugt von 1:0,7 bis 1:1,5. Explizit verwendbare bevorzugte Gewichtsverhältnisse sind: 1:0,50; 1:0,60; 1:0,70; 1:0,75; 1:0,80; 1:0,85; 1:0,90; 1:0,95; 1:1; 1:1,05; 1:1,10; 1:1,20; 1:1,25; 1:1,30; 1:1,40; 1:1,50; 1:1,60; 1:1,70; 1:1,80; 1:1,90. Bei diesen genannten Gewichtsverhältnissen werden die erfindungsgemäßen Effekte besonders wirksam erzielt.

Der pH-Wert der topischen Zusammensetzung beträgt bevorzugt 3,0 bis 8,0, bevorzugt 3,0 bis 5,9, weiter vorzugsweise 3,5 bis 5,4 und besonders bevorzugt 4,0 bis 5,0 (gemessen bei 21 °C mittels pH-Meter, Mettler-Toledo SevenCompact S220). In diesem Bereich sind die erfindungsgemäßen Zusammensetzungen nicht nur chemisch, physikalisch und mikrobiologisch besonders stabil, sondern auch medizinisch und kosmetisch äußerst verträglich für die Haut. Besonders geeignete pH-Werte für die erfindungsgemäße Zusammensetzung sind: 3,5; 3,6; 3,7; 3,8; 3,9; 4,0; 4,1; 4,2; 4,3; 4,4; 4,5; 4,6; 4,7; 4,8; 4,9; 5,0; 5,1; 5,2; 5,3; und 5,4. Der pH-Wert der Zusammensetzung wird bevorzugt eingestellt mithilfe eines oder mehrerer pH-Modifizierungsmittel. Geeignete pH-Modifizierungsmittel sind neben den erfindungsgemäß eingesetzten Carbonsäuren weitere Säuren, Basen und/oder Puffersysteme, die den pH-Wert der Zusammensetzung stabilisieren bzw. beeinflussen. Typische pH-Modifizierungsmittel gemäß der vorliegenden Erfindung sind neben den genannten Carbonsäuren auch deren entsprechenden Salze, sowie Natriumalginat, Polyacrylsäure, Natriumcarbonat und Natriumbicarbonat. Im Zusammenhang mit pH-Modifizierungsmitteln bezieht sich der Begriff Salz auf Alkalimetallsalze oder Erdalkalimetallsalze, sofern nichts anderes angegeben ist.

Die erfindungsgemäße Zusammensetzung enthält Dimethylglycin und/oder ein Salz von Dimethylglycin vorzugsweise in einem Anteil von 0,00001 Gew.-% bis 25,0 Gew.-%, bezogen auf das Gesamtgewicht der Zusammensetzung. In einer bevorzugten Ausführungsform enthält die erfindungsgemäße Zusammensetzung Dimethylglycin und/oder ein Salz von Dimethylglycin in einem Anteil von 0,001 Gew.-% bis 10,0 Gew.- %, bevorzugter von 0,01 Gew.-% bis 8,0 Gew.-%, mehr bevorzugt von 0,1 Gew.-% bis 6,0 Gew.-%, noch mehr bevorzugt von 0,3 Gew.-% bis 5,0 Gew.-%, insbesondere von 0,5 Gew.-% bis 3,0 Gew.-%, jeweils bezogen das Gesamtgewicht der Zusammensetzung. In einer bevorzugten Ausführungsform der Erfindung kann die erfindungsgemäße Zusammensetzung 0,1 Gew.%, 0,2 Gew.%, 0,3 Gew.%, 0,4 Gew.%, 0,5 Gew.%, 0,6 Gew.%, 0,7 Gew.%, 0,8 Gew.%, 0,9 Gew.%, 1,0 Gew.%, 1,1 Gew.%, 1,2 Gew.%, 1,3 Gew.%, 1,4 Gew.%, 1,5 Gew.%, 2,0 Gew.% oder 2,5 Gew.% Dimethylglycin und/oder ein Salz von Dimethylglycin enthalten, jeweils bezogen auf das Gesamtgewicht der Zusammensetzung. Bevorzugt enthält die erfindungsgemäße Zusammensetzung Dimethylglycin und/oder ein Salz von Dimethylglycin als chemischen Reinstoff, einschließlich der jeweiligen Solvate und Hydrate (z.B. des Dihydrats von Natriumdimethylglycinat), da so die Reinheit der Zusammensetzung erhöht und das Auftreten von unerwünschten Nebenwirkungen vermindert werden kann. Aus diesem Grund enthält die erfindungsgemäße Zusammensetzung bevorzugt die chemischen Derivate von Dimethylglycin, ausgewählt aus Methylglycin, Trimethylglycin, (2-Hydroxyethyl)-trimethylammonium, und Trimethyl-hydroxybutyrobetain, in Konzentrationen von weniger als 0,01 Gew.%, bezogen auf das Gesamtgewicht der Zusammensetzung. Besonders bevorzugt sind die erfindungsgemäßen Zusammensetzungen vollständig frei von diesen Derivaten.

Bevorzugt enthält die erfindungsgemäße Zusammensetzung nicht die folgenden Kombinationen:
a) 1,0 Gew.% Na-Dimethylglycinat und 0,2 Gew.% Zitronensäure;
b) 0,4 Gew.% Na-Dimethylglycinat und 0,2 Gew.% Zitronensäure;
c) 0,5 Gew.% Na-Dimethylglycinat und 0,1 Gew.% Milchsäure;
d) 0,4 Gew.% Na-Dimethylglycinat und Zitronensäure (q.s. pH 5,5);
e) 0,4 Gew.% Na-Dimethylglycinat und Glykolsäure (q.s. pH 4,0);
f) 0,4 Gew.% Na-Dimethylglycinat und Milchsäure (q.s. für pH 5,9);
g) 1,0 Gew.% Dimethylglycin HCl und 0,2 Gew.% Zitronensäure;
h) 0,5 Gew.% Dimethylglycin HCl und 0,1 Gew.% Milchsäure.

In einer bevorzugten Ausführungsform enthält die erfindungsgemäße Zusammensetzung mindestens einen weiteren Wirkstoff, wobei der mindestens eine weitere Wirkstoff ausgewählt ist aus Koffein, Menthol, Biotin, Zink PCA, Niacinamid, Panthenol, Ectoin, Ubichinon-10, Taurin, Pantolacton, Echinacea, Tocopherylacetat und Kombinationen davon. Bevorzugt umfasst der Wirkstoff bzw. die Wirkstoffkombination nicht die Verbindungen Methylmethionin, Glycero-Phosphocholin, Phosphatidylcholin, N-Acylethanolamin, und/oder N-Acylphosphatidylethanolamin z.B. mit einem C1-C22-Acylrest.

Alternativ enthält die erfindungsgemäße topische Zusammensetzung gerade kein Koffein. Diese Ausführungsform wird auch als (vollständig) koffeinfreie topische Zusammensetzung bzw. als erfindungsgemäße topische Zusammensetzung ohne Koffein bezeichnet, für die abgesehen davon alle weiteren hierein beschriebenen bevorzugten Merkmale der Erfindung gelten.

Erfindungsgemäß bevorzugt ist weiterhin die Kombination von Dimethylglycin bzw. einem der Salze von Dimethylglycin und einem oder mehreren der folgenden Wirkstoffe.

Menthol ist ein monocyclischer Monoterpenalkohol und kann der erfindungsgemäßen Zusammensetzung als die Durchblutung stimulierender Wirkstoff zugesetzt werden. Zudem kann Menthol eine erfrischende sensorische Stimulierung der Haut bewirken.

Biotin, welches auch als Vitamin B₇ oder Vitamin H bezeichnet wird, ist ein wasserlösliches Vitamin_aus dem B-Komplex. Biotin kann erfindungsgemäß die Haut stärken.

Zink PCA ist das Zinksalz des L-Pyrrolidon-Carboxylats und kann als Substanz mit antimikrobieller Wirkung der erfindungsgemäßen Zusammensetzung zugesetzt werden.

Niacinamid (auch Nicotinamid) ist das Amid der Nicotinsäure und wird auch als Vitamin B₃ bezeichnet. Neben anderen Eigenschaften wie beispielweise einer Verringerung von oxidativem Stress hat das Niacinamid erfindungsgemäß eine die Haut stimulierende Wirkung.

Panthenol ist ein Provitamin, welches im Körper zu Pantothensäure (Vitamin B₅) umgewandelt wird. Letzteres ist Teil des Coenzyms A und damit für den Hautstoffwechsel wichtig. Bei Einwirkung von Panthenol wird erfindungsgemäß die Hautelastizität und Feuchtigkeit weiter verbessert. Daneben werden Juckreiz und Entzündungen gelindert und die Wundheilung gefördert.

Ectoin ist eine zyklische Aminosäure und liegt in wässriger Lösung als mesomeriestabilisiertes Zwitterion vor. Ectoin wirkt befeuchtend und stabilisiert erfindungsgemäß die natürliche Struktur der Haut weiter. Zudem hat sich gezeigt, dass Ectoin vor UV-Strahlung schützt und bei der Behandlung entzündlicher Krankheiten hilfreich sein kann.

Ubichinon-10 (Q10 oder Coenzym Q₁₀) ist ein Chinon-Derivat. Das zum Ubichinon-Pool gehörende Q10 gilt als Antioxidans und wirkt erfindungsgemäß stabilisierend auf die Haut.

Taurin oder 2-Aminoethansulfonsäure wirkt erfindungsgemäß als Antioxidans ebenfalls weiter stabilisierend auf die Haut.

Pantolacton entstammt der Gruppe der substituierten Lactone und regt erfindungsgemäß die Wachstumsfaktoren der Haut weiter an.

Echinacea wirkt erfindungsgemäß beruhigend auf die Haut und lindert auftretenden Juckreiz sowie Spannungen. Zudem kann Echinacea die Blutzirkulation der Haut anregen.

Tocopherylacetat zeigt antioxidative Eigenschaften und wirkt erfindungsgemäß weiter stabilisierend auf die Haut.

In einer bevorzugten Ausführungsform der Erfindung enthält die erfindungsgemäße Zusammensetzung mindestens einen weiteren Wirkstoff ausgewählt aus Menthol, Biotin, Zink PCA, Niacinamid, Panthenol, Ectoin, Ubichinon, Taurin, Pantolacton, Echinacea, Tocopherylacetat und Kombinationen davon jeweils in einem Anteil von 0,001 Gew.-% bis 10,0 Gew.-%, mehr bevorzugt 0,005 Gew.-% bis 7,50 Gew.-%, noch mehr bevorzugt von 0,01 Gew.-% bis 5,0 Gew.-%, insbesondere von 0,1 Gew.-% bis 3,0 Gew.-%, bezogen auf das Gesamtgewicht der Zusammensetzung.

Die erfindungsgemäße Zusammensetzung liegt bevorzugt als Emulsion oder als Gel (Gelformulierung) oder als Mischform aus Emulsion und Gel (Emulgel) vor. Geeignete Emulsionen umfassen mindestens eine wässrige Phase und eine Ölphase (lipophile Phase) sowie ein Emulgatorsystem. Die lipophile Phase beträgt bevorzugt 25 bis 70 Gew.-% der Zusammensetzung (Emulsion), besonders bevorzugt 35 bis 60 Gew.-% und ganz besonders bevorzugt 40 bis 55 Gew.-%. Die wässrige Phase beträgt bevorzugt 20 bis 65 Gew.-% der Zusammensetzung (Emulsion), besonders bevorzugt 30 bis 55 Gew.-% und ganz besonders bevorzugt 35 bis 50 Gew.-%. Das Emulgatorsystem beträgt vorzugsweise 2 bis 10 Gew.%, besonders bevorzugt 4 bis 7 Gew.-% der Zusammensetzung (Emulsion). Die Art der Emulsion ist erfindungsgemäß unkritisch. Erfindungsgemäß geeignet sind Emulsionen vom W/O-, O/W-, W/O/W- und O/W/O-Typ. Erfindungsgemäß geeignete Emulsionen sind dem Fachmann bekannt (z.B. aus der DE102012002950A1 oder DE102010029628A1).

Das bedeutet aber auch, dass die erfindungsgemäße Emulsion bevorzugt keine weiteren Trägersubstanzen aufweist. Bevorzugt enthält die erfindungsgemäße Zusammensetzung keine lamellare Struktur (insbesondere keine Vesikel mit lamellarer Doppelmembranstruktur und/oder Liposome). Sie enthält bevorzugt auch keine lamellare Doppelmembranstrukturen ausbildende Substanz(en).

Geeignete Gelformulierungen der Erfindung enthalten ein Geliermittel (auch Gelbildner genannt). Im Allgemeinen können alle Geliermittel, die im pharmazeutischen und/oder kosmetischen Bereich zur Herstellung stabiler Gelformulierungen bekannt sind, im Rahmen der Erfindung verwendet werden. Vorzugsweise werden hydrophile Geliermittel verwendet. Beispiele für geeignete Geliermittel sind natürliche Geliermittel wie Pektin, Agarose, Gelatine und Casein oder modifizierte natürliche Geliermittel wie Cellulosederivate einschließlich Methylcellulose, Hydroxymethylcellulose, Hydroxyethylcellulose (HEC), Hydroxymethylpropylcellulose (HPMC) und Carboxymethylcellulose, oder vollsynthetische Geliermittel, wie Polyvinylalkohole, Poly(meth)acrylsäuren, Polyacrylamid, Polyvinylpyrrolidon, Polypropylenglykol, Polyethylenglykol und Poloxamere. Cellulosederivate und Poloxamere werden wegen der höheren Stabilität und der angemessenen Viskosität der erhaltenen Gele bevorzugt. Am bevorzugtesten sind Poloxamere aus Gründen der Gelstabilität und wegen ihrer Funktion als Lösungsvermittler. Geeignete Poloxamere sind Poloxamer 407 (z. B. Handelsname Pluronic F 127; Kolliphor P 407), oder Poloxamer 188 (z. B. Handelsname Pluronic F 68; Kolliphor P 188).

Geeignete Gelformulierungen enthalten bis zu 20 Gew.-% lipophile Komponenten, bevorzugt 10 bis 20 Gew.-%, bezogen auf die Gelformulierung. Erfindungsgemäß geeignete Gelformulierungen sind dem Fachmann bekannt.

Geeignete Mischformulierungen aus Emulsion und Gel (Emulgele), die erfindungsgemäß zum Einsatz kommen können, sind dem Fachmann ebenfalls bekannt.

Bevorzugt weist die Zusammensetzung (insbesondere die Emulsion oder das Gel) eine Viskosität von 800 bis 20000 mPa·s auf. Besonders bevorzugte untere Grenzwerte dieses Bereichs betragen 1000 mPa·s, 2000 mPa·s, 3000 mPa·s, 4000 mPa·s, 5000 mPa·s, 6000 mPa·s, 7000 mPa·s, 8000 mPa·s, 9000 mPa·s, und 10000 mPa·s. Besonders bevorzugte obere Grenzwerte dieses Bereichs betragen 19000 mPa·s, 18000 mPa·s, 17000 mPa·s, 16000 mPa·s, 15000 mPa·s, 14000 mPa·s, 13000 mPa·s und 12000 mPa·s. Ganz besonders bevorzugt reicht der Bereich von oberhalb 6000 bis 20000 mPa·s, bevorzugt von 6500 bis 20000 mPa·s und besonders bevorzugt von 7500 bis 15000 mPa·s. Die Viskositätswerte werden jeweils gemessen gemäß DIN 53019-1:2008-09 mit dem Rheometer Haake RheoStress1 (ThermoFisher Scientific) bei 20°C und einer Schergeschwindigkeit von 10/s in Platte-Platte Geometrie (Drehkörper PP60 Ti). In den oben genannten erfindungsgemäßen Viskositätsbereichen erfolgt nicht nur die Freisetzung des Wirkstoffs (d.h. Dimethylglycin und/oder ein Salz von Dimethylglycin) aus der Zusammensetzung besonders deutlich beschleunigt und insgesamt verbessert, sondern auch die Penetration des Wirkstoffs in und durch die Haut verläuft deutlich schneller und vollständiger.

In einer Ausführungsform umfasst die Zusammensetzung ein Tensid. Das Tensid kann ein anionisches, nichtionisches, kationisches oder zwitterionisches Tensid sein. Bevorzugt ist das Tensid ein anionisches oder nichtionisches Tensid, insbesondere ein möglichst mildes (d.h. besonders hautverträgliches) anionisches oder nichtionisches Tensid. Nichtionische Tenside werden dabei erfindungsgemäß insbesondere wegen ihrer sehr guten Emulgationseigenschaften sowie ihrer ausgezeichneten Hautpflegeeigenschaften eingesetzt. Anionische Tenside sind bevorzugt, weil sie eine besonders hohe Reinigungsleistung aufweisen.

Die erfindungsgemäße Zusammensetzung enthält Tenside vorzugsweise in einer Menge von 2 bis 40 Gew.%, insbesondere von 5 bis 30 Gew.%, bevorzugt von 7 bis 20 Gew.%, besonders bevorzugt von 10 bis 17 Gew.%, jeweils bezogen auf das Gesamtgewicht der Zusammensetzung. Geeignete Mengen an Tensid lauten: 8 Gew.%; 9 Gew.%; 10 Gew.%; 11 Gew.%; 12 Gew.%; 13 Gew.%; 14 Gew.%; 15 Gew.%; 16 Gew.%; 17 Gew.%; 18 Gew.%; 19 Gew.%; 20 Gew.%; 21 Gew.%; 22 Gew.%; 23 Gew.%; 24 Gew.%; 25 Gew.%, jeweils bezogen auf das Gesamtgewicht der Zusammensetzung. Besonders bevorzugt enthält die topische Zusammensetzung der Erfindung ein oder mehrere anionische Tenside in einer Menge von 0,1 bis 20 Gew.%, bevorzugt von 1 bis 17 Gew.% und besonders bevorzugt von 5 bis 15 Gew.%, jeweils bezogen auf das Gesamtgewicht der Zusammensetzung. Geeignete Mengen an anionischem Tensid lauten: 1 Gew.%; 2 Gew.%; 3 Gew.%; 4 Gew.%; 5 Gew.%; 6 Gew.%; 7 Gew.%; 8 Gew.%; 9 Gew.%; 10 Gew.%; 11 Gew.%; 12 Gew.%; 13 Gew.%; 14 Gew.%; 15 Gew.%; 16 Gew.%; 17 Gew.%; 18 Gew.%, 19 Gew.%, 20 Gew.%, jeweils bezogen auf das Gesamtgewicht der Zusammensetzung. In diesen Mengen weisen die Tenside eine besonders hohe Reinigungsleistung auf und sind äußerst verträglich für die Haut.

Die Tenside der vorliegenden Erfindung sind u.a. in dem Buch "Surfactants and interfacial phenomena", von Milton Rosen und Joy Kunjappu, John Wiley & Sons, Inc.-Verlag, 2012, 4. Auflage beschrieben.

In einer bevorzugten Ausführungsform ist das Tensid ein anionisches Tensid ausgewählt aus Alkylsulfonaten, Alkylsulfaten, Alkylethersulfaten, Alkylphosphaten, Alkylsarkosinaten, Alkyltauraten, Aminosäure-Tensiden und Mischungen davon. Besonders bevorzugt ist das Tensid ausgewählt aus Alkylsulfaten, Alkylsarkosinaten, Alkyltauraten, Alkylglutamat, wie Natriumcocoylglutamat/Dinatriumcocoylglutamat, Alkylgycinat, Alkylalaninat, wie Natriumcocoylalaninat und Mischungen davon. Ebenso bevorzugt wegen ihrer Reinigungsleistung sind Fettalkoholpolyglycerolethersulfate, Monoglyceridsulfate, Mono- und/oder Dialkylsulfosuccinate, Fettsäureisethionate, und α-Olefinsulfonate.

Alkylsulfate haben die generische Formel ROSO₃M, Alkylsarkosinate haben die generische Formel RC(O)N(CH₃)CH₂CO₂M, und Alkyltaurate haben die generische Formel RC(O)N(CH₃)CH₂CH₂SO₃M, wobei R jeweils ein C₄-C₂₆ Alkyl oder C₄-C₂₆ Alkenyl ist und M ein wasserlösliches Kation wie z.B. Ammonium, Natrium oder Kalium darstellt. Vorzugsweise ist M ein Natriumkation. Bevorzugt ist R ein C₁₂-C₁₆ Alkyl oder ein C₁₂-C₁₈ Alkyl.

In einer Ausführungsform ist das Tensid ein nichtionisches Tensid (auch als nichtionischer Emulgator bezeichnet). Nicht-einschränkende Beispiele umfassen Glycerinfettsäureester, Polyoxyethylenether eines oder mehrerer Fettalkohole, alkoxylierte Fettsäurealkylester, Polyglycerinether von Fettalkoholen, Polyglycerinester von Fettsäuren, Polyethylenglykol- und/oder Polypropylenglykolether, Fettsäureamide, Alkylphenolpolyglycolether, Aminoxide und Alkylpolyglucoside.

In einer Ausführungsform ist das Tensid ausgewählt aus der Gruppe der Glycerinfettsäureester, Polyoxyethylenether eines oder mehrerer Fettalkohole, Polyglycerinether von Fettalkoholen, Polyglycerinester von Fettsäuren und deren Mischungen.

In der vorliegenden Erfindung bezieht sich der Begriff "Glycerinfettsäureester" auf einen Glycerinmono- oder Glycerindifettsäureester. Glycerindifettsäureester weisen die Formel R³-COO-(CH₂CH(OH)CH₂)-OOR⁴ oder R³-COO-(CH₂CH(OOR⁴)CH₂)-OH auf. Glycerinmonofettsäureester weisen die Formel R³-COO-(CH₂CH(OH)CH₂)-OH oder HO-(CH₂CH(OOR³)CH₂)-OH auf. Dabei sind R³ und R⁴ unabhängig voneinander aus C₆-C₂₈ Alkyl und C₆-C₂₈ Alkenyl ausgewählt. Glycerinmonofettsäureester enthalten eine Glycerin-Gruppe, welche über eine Esterbindung an eine einzige Fettsäure gebunden. Beispiele sind Glycerinmonostearat, Glycerinmonobehenat, Glycerinmonocaprylat, Glycerinmonocaprat und Glycerinmonolaurat.

Polyoxyethylenether sind Verbindungen der Formel R⁵(OC₂H₃)ₙOH, wobei R⁵ ausgewählt ist aus C₆-C₂₈ Alkyl, C₆-C₂₈ Alkenyl, substituierte und unsubstituierte Phenoxy-Gruppen; und n eine ganze Zahl größer 1 ist. Vorzugsweise wird der Polyoxyethylenether eines oder mehrerer Fettalkohole aus der Gruppe Steareth-2, Steareth-21, Makrogol-Cetostearylether 12, Ceteareth-25, Makrogol-Cetostearylether 20 und Mischungen der vorgenannten Verbindungen ausgewählt. Noch bevorzugter ist der Polyoxyethylenether eine Verbindung ausgewählt aus der Gruppe von Ceteareth-25, Makrogol-Cetostearylether 20 und Mischungen der vorgenannten Verbindungen.

Der Begriff "Polyglycerinether von Fettalkoholen" bezieht sich auf eine Verbindung der Formel R⁶O-(C₃H₆O₂)ₙ-H, wobei R⁶ ein verzweigtes oder lineares C₆-C₂₈ Alkyl oder C₆-C₂₈ Alkenyl ist und n eine ganze Zahl größer als 1, vorzugsweise eine ganze Zahl von 2 bis 10, ist. Es wird bevorzugt, dass die Zusammensetzung 0,01 bis 15,0 Gew.-%, 0,1 bis 10,0 Gew.-% oder 1 bis 5,0 Gew.-% Polyglycerinether enthält.

Der Begriff "Polyglycerinester von Fettsäuren" bezieht sich auf Verbindungen, die sowohl eine Polyglycerineinheit als auch mindestens eine C₆-C₂₆ Alkyl- oder C₆-C₂₆ Alkenylcarbonsäureeinheit enthalten. Diese Verbindungen können die Formel R⁷-R⁸-(C₃H₆O₂)ₙ-H aufweisen, wobei R⁷ ein C₆-C₂₆ Alkanoat- oder C₆-C₂₆ Alkenoat-Rest ist und R⁸ ein geeignetes Verbindungsmolekül oder eine direkte Bindung ist. Somit können die Polyglycerineinheit und die C₆-C₂₆ Alkyl- oder C₆-C₂₆ Alkenylcarbonsäureeinheit direkt durch eine Esterbindung verbunden sein oder eine Verbindungseinheit enthalten, die diese beiden Einheiten miteinander verbindet. Nicht einschränkende Beispiele für diese Gruppe sind Polyglyceryl-3-methylglucosedistearat, Polyglycerinpolycrinoleat, Polyglyceryl-Dimerat-Isostearat, Polyglyceryl-2-Laurat, Polyglyceryl-2-Sesquiisostearat, Polyglyceryl-3-Distearat (Cremophor GS 32), Polyglyceryl-3-Oleat, Polyglyceryl-3-Methylglykose-Distearat, Polyglyceryl-4-Caprat (Polyglycerolcaprat T2010190), Polyglyceryl-4-Diisostearat / Polyhydroxystearat / Sebacat (Isolan GPS) und Polyglyceryl-4-Isostearat.

In einer Ausführungsform umfasst das Tensid ein kationisches Tensid, wie beispielsweise ein quaternäres Tensid. Quaternäre Tenside enthalten mindestens ein N-Atom, das mit 4 Alkyl- oder Arylgruppen kovalent verbunden ist. Dies führt, unabhängig vom pH-Wert, zu einer positiven Ladung. Vorteilhaft sind Alkylbetain, Alkylamidopropylbetain und Alkylamidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

In einer bevorzugten Ausführungsform ist die erfindungsgemäße Zusammensetzung eine Ölin-Wasser-Emulsion (O/W), in der die vorstehend beschriebenen Tensidkomponenten (auch als Emulgatorkomponenten bezeichent) einen HLB-Wert von 8-40, bevorzugt von 8-22, besonders bevorzugt von 8-18 aufweisen. In einer anderen bevorzugten Ausführungsform ist die erfindungsgemäße Zusammensetzung eine Wasser-in-Öl-Emulsion (W/O), in der die vorstehend beschriebenen Tensidkomponenten (auch als Emulgatorkomponenten bezeichent) einen HLB-Wert von 3-8, bevorzugt von 4-5 aufweisen. Das Berechnungsverfahren für den kombinierten HLB-Wert ist bekannt und kann als Griffin- oder Davies-Verfahren bezeichnet werden (Ullmann's Encyclopedia of Industrial Chemistry, Teil 9, Emulsion, 2005; Surfactant Application Encyclopedia, Liu Cheng, Beijing Industry Press, 1997).

In einer weiteren Ausführungsform enthält die erfindungsgemäße Zusammensetzung mindestens ein Additiv. Bevorzugt sind Additive, die üblicherweise in Emulsionen zur Behandlung der Haut eingesetzt werden. Das mindestens eine Additiv kann in einem Anteil von 0,01 Gew.-% bis 12,0 Gew.-%, mehr bevorzugt von 0,25 Gew.-% bis 10,0 Gew.-%, insbesondere von 1,0 bis 7,0 Gew.-% vorliegen.

Das mindestens eine Additiv kann ferner ausgewählt sein aus der Gruppe bestehend aus Rückfettern, Konservierungsmitteln, Stabilisatoren, Duftstoffen, Antioxidantien, Rheologiemodifikatoren, Verdickungsmitteln, Pflegemitteln, Farbstoffen, Perlganzmitteln, Aufhellungsmitteln, Lösungsmitteln, Sonnenschutzmittel und Kombinationen hiervon.

Rückfetter, auch Rückfettungsmittel oder Überfettungsmittel genannt, sind lipophile Substanzen, die eine störende Auswirkung auf die epidermale Barrierefunktion verhindern können. Beispiele für Rückfetter sind Wollwachs, Squalen, flüssiges Paraffin, pflanzliche Öle, Silikone und Cetylpalmitat.

Konservierungsmittel sind Substanzen, die zur Konservierung verwendet werden, indem sie die Zusammensetzung zersetzende Mikroorganismen abtöten und/oder deren Wachstum hemmen. Vorzugsweise können die Konservierungsmittel ausgewählt sein aus der Gruppe bestehend aus Benzoesäure, Benzoesäurederivaten, Sorbinsäure, Sorbinsäurederivaten, Salicylsäure, Salicylsäurederivaten, Phenoxyethanol, Parabenen und Kombinationen hiervon. In einer bevorzugten Ausführungsform werden Natriumbenzoat und/oder Kaliumsorbat als Konservierungsmittel in der erfindungsgemäßen Zusammensetzung eingesetzt. Natriumbenzoat setzt in leicht saurem Milieu Benzoesäure und Kaliumsorbat Sorbinsäure frei. Beiden Säuren wird eine antimikrobielle Wirkung zugeschrieben.

Stabilisatoren können lichtempfindliche Komponenten gegenüber Strahlung schützen und sind bevorzugt UV-Absorber wie beispielsweise Benzophenonderivate.

Die Zugabe von Duftstoffen kann für einen angenehmen Geruch der Zusammensetzung sorgen. Beispiele sind die dem Fachmann bekannten Parfums.

Ein Antioxidans oder Antioxidationsmittel ist eine chemische Verbindung, die eine Oxidation anderer Komponenten in der erfindungsgemäßen Zusammensetzung verlangsamt oder gänzlich verhindert. Als Antioxidantien kommen beispielsweise Zitronensäure, Ascorbinsäure und Butylhydroxyanisol in Frage.

Rheologiemodifikatoren und Verdickungsmittel können dabei helfen, die Applikationseigenschaften der erfindungsgemäßen Zusammensetzung zu verbessern. Als Rheologiemodifikator und Verdickungsmittel kann die Zugabe von Kochsalz (Natriumchlorid) in Betracht gezogen werden. Durch die Zugabe von Kochsalz kann die Fließfähigkeit der erfindungsgemäßen Zusammensetzung in gewissen Grenzen beeinflusst und auf das nötige Maß eingestellt werden. Als Verdicker können zusätzlich natürlich vorkommende Gelbildner eingesetzt werden, die bevorzugt ausgewählt sind aus Agar, Xanthan Gum, Cellulose und oder Cellulose-Derivaten oder Alginsäure.

Im Rahmen der Anmeldung sollen unter Pflegemitteln Substanzen verstanden werden, welche die Haut pflegen. Hydrolysiertes Weizenprotein und Allantoin wirken pflegend die Haut. Hydrolysiertes Weizenprotein hat hauptsächlich feuchtigkeitsspendende Eigenschaften.

Farbstoffe werden optional dazu verwendet, der erfindungsgemäßen Zusammensetzung eine charakteristische Farbe zu verleihen, so dass diese leicht von anderen Produkten unterschieden werden kann.

Als Lösungsmittel kann ein für den Fachmann auf diesem Gebiet gängiges Lösungsmittel oder Lösungsmittelgemisch eingesetzt werden. Bevorzugte Lösungsmittel sind Ethanol oder Butylenglykol, insbesondere 1,4-Butylenglykol, Propylenglykol und Isopropylalkohol. Bevorzugt ist Ethanol. Diese Lösungsmittel können vorzugsweise in einer Menge von 0,1 bis 70 Gew.% in der erfindungsgemäßen Zusammensetzung enthalten sein. Ganz besonders bevorzugt sind sie in einer Menge von 0,1 bis weniger als 5,0 Gew.% enthalten. Außerdem helfen Lösungsmittel bei der Penetration der Wirkstoffe und verstärken somit deren Wirksamkeit.

Geeignete Sonnenschutzmittel sind UVA-Filtersubstanzen und/oder UVB-Filtersubstanzen und/oder anorganische Pigmente.

Vorteilhaft können erfindungsgemäße Emulsionen Substanzen enthalten, die UV-Strahlung im UVB- Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Emulsion beträgt.

Die UVB-Filter können öllöslich oder wasserlöslich sein. Als öllösliche Substanzen sind z. B. zu nennen:
- 3-Benzylidencampher und dessen Derivate, z. B. 3-(4-Methylbenzyliden)campher,
- 4-Aminobenzoesäurederivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Metboxyzimtsäureisopentylester;
- Ester der Salicylsäure, vorzugsweise Salicylsäure(2-ethylhexyl)ester, Salicylsäure.(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'- Dihydroxy-4-metboxybenzophenon;
- Ester der Benzalmalonsäure, vorzugsweise 4-Methoxybenzalmalonsäuredi(2-ethylhexyl)ester;- 2,4,6- Trianilino-(p-carbo-2'-ethyl-1'-hexyloxy)-1,3,5-triazin.

Als wasserlösliche Substanzen sind vorteilhaft:
2-Phenylbenzimidazol-5-sulfonsäure und deren Salze, z. B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsaure-Derivate des 3-Benzylidencamphers, wie z.B. 4-(Oxo-3-bornylidenmethyl)benzolsulfonsäure, 2-Methyl-5-(2-oxo-3-bornylidenmethyl)sulfonsäure und ihre Salze.

Die erfindungsgemäßen Emulsionen können auch UVA-Filter enthalten. Bei solchen Filtersubstanzen handelt es sich vorzugsweise um Derivate des Dibenzoylmethans, insbesondere um 1-(4'-tert.Butylphenyl)-3-(4'-methoxyphenyl)-propan-1,3-dion und um 1-Phenyl-3-(4-isopropylphenyl)propan-1,3-dion. Auch Emulsionen, die Kombinationen der genannten Filter enthalten, sind Gegenstand der Erfindung. Es können die gleichen Mengen an UVA-Filtersubstanzen verwendet werden, welche für UVB-Filtersubstanzen genannt wurden.

Die erfindungsgemäßen Emulsionen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon. Besonders bevorzugt handelt es sich um Pigmente auf der Basis von Titandioxid. Es können die für die vorstehenden Kombinationen genannten Mengen verwendet werden.

Erfindungsgemäß wird die Zusammensetzung topisch angewendet. Unter einer topischen Anwendung ist eine äußerliche Anwendung, insbesondere eine örtliche, äußerliche Anwendung zu verstehen. Erfindungsgemäße Zusammensetzungen sind bevorzugt Hautpflegeprodukte (d.h. Behandlungsmittel wie Kuren, Lotionen, Duschbäder, Tages- oder Nachtcreme, Gesichtscreme, Gesichtsfluid, Gesichtsmaske, Hautschutzcreme, Reinigungsmittel, Sonnenschutzlotionen, desodorierende Zusammensetzungen, Rasiercreme, After Shave-Balsam, und/oder ein Tonikum) und keine (reinen) Styling-Produkte. Mit anderen Worten liegt bei den topischen Zusammensetzungen der Erfindung der Fokus auf der medizinischen/pharmazeutischen oder kosmetischen Behandlung der Haut.

Die vorliegende Erfindung bezieht sich weiterhin auf die Verwendung der erfindungsgemäßen Zusammensetzung zur Behandlung der Haut. Die Haut ist erfindungsgemäß besonders bevorzugt die Körperhaut, einschließlich des Oberkörpers und des Unterkörpers, sowie einschließlich der Hände, Arme, Beine und Füße, und/oder die Gesichtshaut, einschließlich des Halses und des Nackens. Die Haut umfasst bevorzugt gerade nicht die Kopfhaut, d.h. die Haut unter dem Haupthaar.

In einer bevorzugten Ausführungsform betrifft die Behandlung der Haut die rein optischästhetische Verbesserung, wie die Schaffung eines glatteren und schlicht schöneren Hautbilds. Erfindungsgemäß werden aber besonders bevorzugt auch darüber hinaus gehende kosmetische/medizinische Effekte erzielt, wie eine verbesserte Schutzfunktion und eine gestärkte Barriereeigenschaft. Zudem wird die Wundheilung verbessert.

Anders ausgedrückt führt die erfindungsgemäße Verwendung zu einer deutlichen Verbesserung der epidermalen Barrierefunktionen und der epidermalen Barriereintegrität, zur Verbesserung des Erscheinungsbildes der Haut und der Erhöhung des Feuchtigkeitsgehalts der Haut. Das geht einher mit einer Steigerung der Kohäsion des Stratum corneum und der Homöostase der Hautbarriere und resultiert schließlich in einem verbesserten Schutz vor Infektionen (mikrobiellen Erkrankungen).

Bei medizinischer Verwendung ist erfindungsgemäß sowohl die therapeutische als auch die prophylaktische Behandlung von Hauterkrankungen umfasst. Bei den Erkrankungen handelt es sich bevorzugt um mikrobielle Hautinfektionen, Hautentzündungen, raue Haut, trockene Haut, Hautirritationen, Juckreiz, Pruritus, Allergien, Psoriasis, psoriatische Arthritis, Ekzeme, Scleroderma, atopische Dermatitis, Kontaktdermatitis, systemischen Lupus erythematosus, Akne und Anfälligkeit gegenüber Kontaktallergien.

Die nicht-therapeutische (d.h. rein kosmetische) Verwendung umfasst insbesondere die Behandlung von kosmetischen Indikationen der Haut, insbesondere ausgewählt aus rauer Haut, trockener Haut, Hautirritationen, Juckreiz und Pruritus, sowie die Vorbeugung von Hautinfektionen und die Verminderung der Anfälligkeit gegenüber Kontaktallergien.

Die erfindungsgemäßen Emulsionen sind insbesondere wirksam in der Verbesserung der epidermalen Barrierefunktionen und der epidermalen Barriereintegrität, bei der Förderung der Wundheilung, in der Behandlung und/oder Prophylaxe von entzündlichen Zuständen der Haut, in der Behandlung und/oder Prophylaxe von Störungen, die mit einer mangelnden Regeneration der Epidermis verbunden sind, sowie in der Verbesserung des Erscheinungsbildes der Haut, der Erhöhung des Feuchtigkeitsgehalts der Haut, der Förderung des natürlichen Hautschutzes und/oder der Vitalisierung der Haut.

Anhand der nachfolgenden Zusammensetzungen soll die Erfindung verdeutlicht werden, ohne sie jedoch auf die speziellen Beispiele einschränken zu wollen.

### Experimenteller Teil

Die folgenden Zusammensetzungen wurden durch für den Fachmann bekannte Homogenisierungsmaßnahmen hergestellt. Die Menge der Komponenten wurde jeweils so gewählt, dass ihr Gewichtsanteil in der fertigen Zusammensetzung angegebenen Gewichtsanteilen entspricht.

### Beispiel 1

Zusammensetzung in Form einer Emulsion, welche die folgenden Komponenten enthält:

| | |
|---|---|
| Zink-Stearat | 1,25 Gew.% |
| Magnesium-Sulfat | 0, 5 Gew.% |
| Sorbitan Isostearate & Polyglyceryl-3 Polyricinoleate | 4,5 Gew.% |
| Hexyldecanol & Hexyldecyllaurat | 25,0 Gew.% |
| Glycerin | 2,0 Gew.% |
| Zitronensäure | 0,4 Gew.% |
| Natrium-Dimethylglycinat | 0,4 Gew.% |
| Parfum | 0,2 Gew.% |
| Konservierungsmittel | q.s. |
| Wasser | ad 100 Gew.% |

### Beispiel 2

Zusammensetzung in Form einer Emulsion, welche die folgenden Komponenten enthält:

| | |
|---|---|
| Cetearyl Ethylhexanoate | 20,0 Gew.% |
| Carbomer | 0,2 Gew.% |
| Polyglyceryl-3 Methylglucose Distearate | 3,0 Gew.% |
| Cetearylalkohol & Natriumcetearylsulfat | 2,0 Gew.% |
| Glycerylstearat | 1,5 Gew.% |
| Zitronensäure | 0,4 Gew.% |
| Natrium-Dimethylglycinat | 0,6 Gew.% |
| Parfum | 0,2 Gew.% |
| Konservierungsmittel | q.s. |
| Wasser | ad 100 Gew.% |

### Beispiel 3

Zusammensetzung in Form einer Emulsion (Viskosität 7500 mPas), welche die folgenden Komponenten enthält:

| | |
|---|---|
| Zitronensäure | 0,83 Gew.% |
| Natrium-Dimethylglycinat | 1,0 Gew.% |
| Phenoxyethanol | 1,00 Gew.% |
| Caprylic/Capric Triglyceride | 20,00 Gew.% |
| Steareth-2 | 3,00 Gew.% |
| Steareth-21 | 3,00 Gew.% |
| Xanthan Gum | 2,00 Gew.% |
| Wasser | ad 100 Gew.% |

### Beispiel 4

Zusammensetzung in Form einer Emulsion (Viskosität 32000 mPas), welche die folgenden Komponenten enthält:

| | |
|---|---|
| Zitronensäure | 0,85 Gew.% |
| Natrium-Dimethylglycinat | 1,0 Gew.% |
| Phenoxyethanol | 1,0 Gew.% |
| Caprylic/Capric Triglyceride | 14,0 Gew.% |
| Steareth-2 | 3,0 Gew.% |
| Steareth-21 | 3,0 Gew.% |
| Xanthan Gum | 1,0 Gew.% |
| Cetearylalkohol | 6,0 Gew.% |
| Wasser | ad 100 Gew.% |

### Beispiel 5

Zusammensetzung in Form einer Emulsion, welche die folgenden Komponenten enthält:

| | |
|---|---|
| Caprylic/Capric Triglyceride | 20 Gew.% |
| Steareth-2 | 3 Gew.% |
| Steareth-21 | 3 Gew.% |
| Xanthan Gum | 2 Gew.% |
| Natrium-Dimethylglycinat | 1 Gew.% |
| Zitronensäure | 0,8 Gew.% |
| Parfümöl | q.s. |
| Konservierungsmittel | q.s. |
| Wasser | ad 100 Gew.% |

### Beispiel 6

Zusammensetzung in Form eines Gels, welche die folgenden Komponenten enthält:

| | |
|---|---|
| Natrium-Dimethylglycinat | 1,0 Gew.% |
| Zitronensäure wasserfrei | 0,83 Gew.% |
| Ethanol | 10,0 Gew.% |
| Poloxamer 407 | 18,0 Gew.% |
| Wasser | ad 100 Gew.% |

### Beispiel 7

Zusammensetzung in Form einer Emulsion, welche die folgenden Komponenten enthält:

| | |
|---|---|
| Caprylic/Capric Triglyceride | 20 Gew.% |
| Cetylalkohol | 2,7 Gew.% |
| Polyglyceryl-3 Methylglucose Distearate | 3,5 Gew.% |
| Natrium-Cetearylsulfat | 0,3 Gew.% |
| Natrium-Dimethylglycinat | 1,0 Gew.% |
| Zitronensäure | 0,7 Gew.% |
| Parfümöl | q.s. |
| Konservierungsmittel | q.s. |
| Wasser | ad 100 Gew.% |

### Referenzbeispiel 1

Zusammensetzung in Form einer Emulsion (mit anorganischer Säure), welche die folgenden Komponenten enthält:

| | |
|---|---|
| Caprylic/Capric Triglyceride | 20 Gew.% |
| Cetylalkohol | 2,7 Gew.% |
| Polyglyceryl-3 Methylglucose Distearate | 3,5 Gew.% |
| Natrium-Cetearylsulfat | 0,3 Gew.% |
| Natrium-Dimethylglycinat | 1,0 Gew.% |
| Salzsäure | 0,7 Gew.% |
| Parfümöl | q.s. |
| Konservierungsmittel | q.s. |
| Wasser | ad 100 Gew.% |

### Referenzbeispiel 2

Zusammensetzung in Form einer Emulsion (ohne Na-Dimethylglycinat), welche die folgenden Komponenten enthält:

| | |
|---|---|
| Caprylic/Capric Triglyceride | 20 Gew.% |
| Cetylalkohol | 2,7 Gew.% |
| Polyglyceryl-3 Methylglucose Distearate | 3,5 Gew.% |
| Natrium-Cetearylsulfate | 0,3 Gew.% |
| Zitronensäure | 0,7 Gew.% |
| Parfümöl | q.s. |
| Konservierungsmittel | q.s. |
| Wasser | ad 100 Gew.% |

### Referenzbeispiel 3

Zusammensetzung in Form einer Emulsion (ohne Na-Dimethylglycinat), welche die folgenden Komponenten enthält:

| | |
|---|---|
| Wasser | ad 100 Gew% |
| Zitronensäure | 0,83 Gew% |
| Natronlauge 25%ig | 1,35 Gew% |
| Phenoxyethanol | 1,00 Gew% |
| Caprylic/Capric Triglyceride | 14,0 Gew% |
| Steareth-2 | 3,0 Gew% |
| Steareth-21 | 3,0 Gew% |
| Xanthan Gum | 1,0 Gew% |
| Cetylstearylalkohol | 6,0 Gew% |

### Referenzbeispiel 4

Zusammensetzung in Form einer Emulsion (ohne Na-Dimethylglycinat), welche die folgenden Komponenten enthält:

| | |
|---|---|
| Caprylic/Capric Triglyceride | 20 Gew.% |
| Steareth-2 | 3 Gew.% |
| Steareth-21 | 3 Gew.% |
| Xanthan Gum | 2 Gew.% |
| Zitronensäure | 0,8 Gew.% |
| Parfümöl | q.s. |
| Konservierungsmittel | q.s. |
| Wasser | ad 100 Gew.% |

### Studiendesign und -ergebnisse

1. Im Rahmen einer *in vitro* Untersuchung wurde die Wirkung von Na-Dimethylglycinat im Zellkulturmodell mit humanen hornbildenden Keratinozyten-Zellen untersucht. Hierfür wurden HaCaT-Zellen für 1, 3, 5, und 7 Tage in DMEM-Medium (inklusive fötalem Kälberserum und einem Antibiotika-Antimykotika-Mix) kultiviert und u.a. die Viabilität, Proliferation und Migration der Zellen durch geeignete Messmethoden bestimmt sowie die Expression der für das Zellwachstum relevanten Wachstumsfaktoren bestimmt.

### Nachweis der Viabilität:

Für diese Messung wurde ein sogenannter MTT-Assay verwendet, um die zelluläre Stoffwechselaktivität als Indikator für die Lebensfähigkeit und Zytotoxizität der Zellen zu bestimmen. Dieser kolorimetrische Assay basiert auf der Reduktion eines gelben Tetrazoliumsalzes (3-(4,5-Dimethylthiazol-2-yl)-2,5-diphenyltetrazoliumbromid oder MTT) zu violetten Formazan-Kristallen durch metabolisch aktive Zellen.

Humane epidermale Keratinozyten-Zellen (HaCaT) wurden in einer 96-Well Platte mit einer Zelldichte von 5.000 Zellen/Well ausgesät und in Medium (DMEM mit 10% FBS, 1% Penicillin-Streptomycin, 0.5% Fungizone) kultiviert. Am nächsten Tag sowie nach weiteren 24 h sowie 48 h und 72 h Kultivierung bei 37°C und 5 Vol.-% CO₂ wurde das Zellkulturmedium ohne (Kontrolle) bzw. mit den jeweils bereits enthaltenen Wirkstoff-Konzentrationen von Natrium-Dimethylglycinate (DMG) gewechselt. Die Messung erfolgte analog zu bereits publizierten Untersuchungen in B. I. Tóth, N. Dobrosi, A. Dajnoki, G. Czifra, A. Oláh, A. G. Szöllösi, I. Juhász, K. Sugawara, R. Paus, T. Bíró, J Invest Dermatol 2011, 131, 1095-1104.

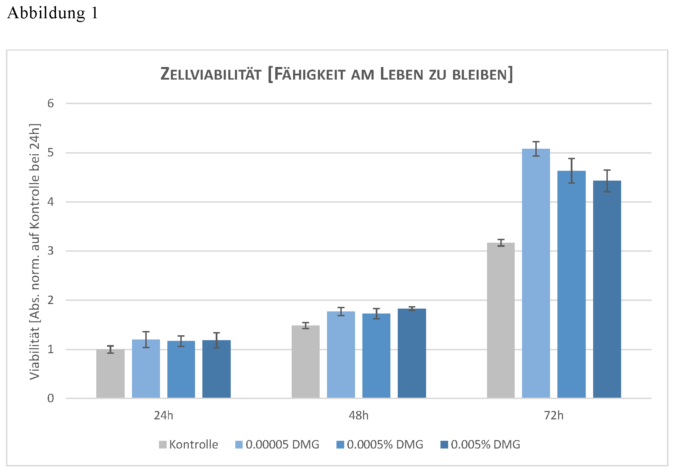

In Abbildung 1 ist die Zellviabilität nach 24 h, 48 h und 72 h Kultivierung dargestellt und eine deutliche Steigerung durch die Zugabe von DMG nach 48 h und 72 h zu sehen. Die Zellviabilität wurde bestimmt mittels MTT-Assay. Die Absorption wurde jeweils in Vierfachbestimmung gemessen und auf die Kontrolle bei 24 h normiert. Angegeben ist der Mittelwert mit Standardabweichung.

### Nachweis der Proliferation:

Für die Messung der Proliferation wurde ein sogenannter CyQUANT-Assay durchgeführt. Bei diesem fluoreszenz-basierten Assay bindet der verwendete Fluoreszenzfarbstoff an DNA (engl. deoxyribonucleic acid), wobei der Gehalt an zellulärer DNA ein direktes Maß für die Anzahl an Zellen innerhalb einer Probe ist.

Humane epidermale Keratinozyten-Zellen (HaCaT) wurden in einer 96-Well Platte mit einer Zelldichte von 5.000 Zellen/Well ausgesät und in Medium (DMEM mit 10% FBS, 1% Penicillin-Streptomycin, 0.5% Fungizone) kultiviert. Am nächsten Tag sowie nach weiteren 24 h sowie 48 h und 72 h Kultivierung bei 37°C und 5 Vol.-% CO₂ wurde das Zellkulturmedium ohne (Kontrolle) bzw. mit den jeweils bereits enthaltenen Wirkstoff-Konzentrationen gewechselt. Die Messung erfolgte analog zu bereits publizierten Untersuchungen in A. Oláh, B. I. Tóth, I. Borbíró, K. Sugawara, A. G. Szöllösi, G. Czifra, B. Pál, L. Ambrus, J. Kloepper, E. Camera, The Journal of clinical investigation 2014, 124, 3713-3724.

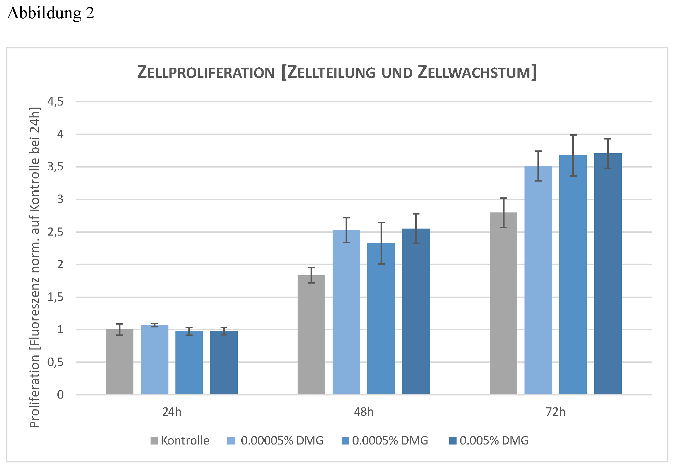

In Abbildung 2 ist die Zellproliferation nach 24 h, 48 h und 72 h Kultivierung dargestellt und eine deutliche Steigerung durch die Zugabe von DMG nach 48 h und 72 h zu sehen. Die Zellproliferation wurde bestimmt mittels CyQUANT-Assay. Die Fluoreszenz wurde jeweils in Dreifachbestimmung gemessen und auf die Kontrolle bei 24 h normiert. Angegeben ist der Mittelwert mit Standardabweichung.

### Nachweis der Migration:

Für die Messung der Migration wurde ein sogenannter *Wound Healing-Assay* durchgeführt, basierend auf bereits publizierten Untersuchungen in T. Kawabata, T. Otsuka, K. Fujita, G. Sakai, R. Matsushima-Nishiwaki, O. Kozawa, H. Tokuda, International journal of molecular medicine 2018, 42, 3149-3156. Das Prinzip basiert darauf, dass die Migration von Zellen auf eine noch nicht besiedelte Kultivierungsoberfläche im Zeitverlauf gemessen wird. Hierfür wurden jeweils 20.000 Zellen in zwei benachbarte und durch einen Silikoneinsatz (mit standardisierter Breite) getrennte Wells bzw. Kavitäten ausgesät und in Medium (DMEM mit 5% FBS, 1% Penicillin-Streptomycin, 0.5% Fungizone) für 48 h bei 37°C und 5 Vol.-% CO₂ kultiviert. Im Anschluss wurde der Plastikeinsatz entfernt ("Erzeugung der Wunde") und die Migration der Zellen durch die Bestimmung der nicht mit Zellen besiedelten Kultivierungsoberfläche im Zeitverlauf durch Bildaufnahmen dokumentiert. Parallel dazu wurde das Zellkulturmedium ohne (Kontrolle) bzw. mit den jeweils bereits enthaltenen Wirkstoff-Konzentrationen von DMG direkt nach Entfernung des Plastikeinsatzes (0 h) sowie nach weiteren 24 h gewechselt. Die Auswertung der BIldaufnahmen bzw. Bestimmung der nicht mit Zellen besiedelten Kultivierungsoberfläche erfolgte über eine bestimmte Software (ImageJ).

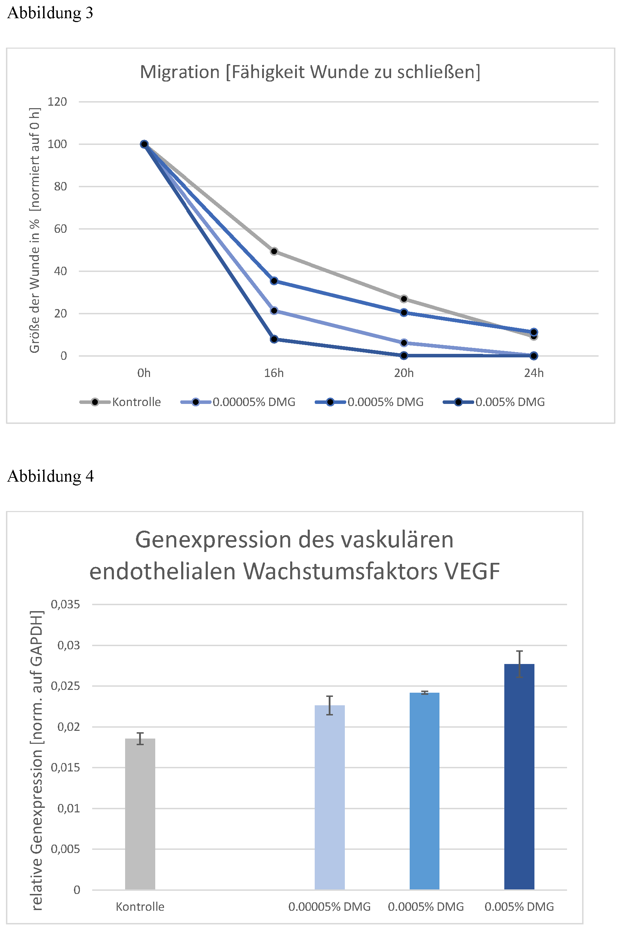

In Abbildung 3 ist die Migration im Zeitverlauf und unter Einsatz der unterschiedlichen DMG-Konzentrationen dargestellt. Die Migration bzw. Wundschließung erfolgt mit DMG jeweils deutlich schneller im Vergleich zur Kontrolle ohne DMG. Zum Zeitpunkt 0 h wurde die Wunde erzeugt und Bildaufnahmen nach 16 h, 20h bzw. 24 h Kultivierung erstellt. Aus Basis der Bildaufnahmen wurde die Kultivierungsoberfläche, welche noch nicht mit Zellen besiedelt war, mithilfe der ImageJ-Software bestimmt. Die Messwerte sind jeweils auf den Zeitpunkt 0 h (maximale Größe der Wunde) normiert und in % angegeben.

### Nachweis der Genexpression von VEGF:

VEGF (engl. *Vascular Endothelial Growth Factor)* fördert das Wachstum und die Bildung neuer Blut- und Lymphgefäße. Die Messung der Genexpression erfolgte über ein StandardVerfahren der sogenannten quantitative Echtzeit-PCR (qRT-PCR).

Humane epidermale Keratinozyten-Zellen (HaCaT) wurden in einer 6-Well Platte mit einer Zelldichte von 140.000 Zellen/Well ausgesät und in Medium (DMEM mit 10% FBS, 1% Penicillin-Streptomycin, 0.5% Fungizone) bei 37°C und 5 Vol.-% CO₂ kultiviert. Am nächsten Tag wurde das Zellkulturmedium ohne (Kontrolle) bzw. mit den jeweils bereits enthaltenen Wirkstoff-Konzentrationen gewechselt und die Zellen nach 24 h geerntet. Die Messung der Genexpression erfolgte mittels qRT-PCR basierend auf bereits publizierten Untersuchungen in B. V. Diaz, M.-C. Lenoir, A. Ladoux, C. Frelin, M. Démarchez, S. Michel, Journal of Biological Chemistry 2000, 275, 642-650.

In Abbildung 4 ist die Genexpression von VEGF nach 24 h Kultivierung dargestellt und eine deutliche Steigerung der Genexpression durch die Zugabe von DMG zu sehen. Dargestellt ist die mittels qRT-PCR ermittelte relative Genexpression (Dreifachbestimmung, normiert auf die jeweilige Genexpression eines konstitutiv exprimiertes Gens; GAPDH - Glycerinaldehyd-3-phosphat-Dehydrogenase) von VEGF. Angegeben ist der Mittelwert mit Standardabweichung.

Es hat sich überraschend gezeigt, dass mit DMG die für das Wachstum relevanten Parameter der HaCaT-Zellen positiv beeinflußt wurden und die Expression des Wachstumsfaktors VEGF signifikant gesteigert wurde gegenüber der Behandlung von HaCaT-Zellen ohne DMG.

2. Die erfindungsgemäße Creme aus Beispiel 7 sowie die Hautcreme aus dem Referenzbeispiel 1 (mit anorganischer Säure) und 2 (ohne Na-Dimethylglycinat) wurden im Rahmen einer kontrollierten und verblindeten Hautfeuchtigkeitsmessung nach Einmalapplikation am Unterarm an 4 Frauen und Männern (Phototyp Fitzpatrick I-III, Alter: 20-64 Jahre) angewendet. Vor Produktanwendung wurden 4 randomisierte Areale am Unterarm definiert und Baseline-Werte der Haut-Feuchtigkeit (mittels Corneometer, Derma Unit SC 2 von Courage + Khazaka Electronic GmbH, Deutschland, bei 22 °C Raumtemperatur) gemessen. Eine Menge von ca. 2 mg/cm² wurde vom jeweiligen Produkt auf das entsprechende Areal am Unterarm aufgetragen und für ca. 30 Sekunden einmassiert, wobei ein Areal als Kontrollfeld unbehandelt blieb. Nach 30, 60, 120 und 180 Minuten erfolgte die Messung der Hautfeuchtigkeit.

Es hat sich gezeigt, dass die Hautfeuchtigkeit deutlich gesteigert wurde durch die Auftragung der erfindungsmäßen Creme im Vergleich zu den beiden Referenzbeispielen und dem unbehandelten Kontrollfeld.

3. Die erfindungsgemäße Creme aus Beispiel 7 sowie die Hautcreme aus dem Referenzbeispiel 1 (mit anorganischer Säure) wurden im Rahmen Osmolalitätsmessung untersucht. Die Osmolalität ist ein Maß für die Gesamtzahl der gelösten chemischen Einheiten und zeigt somit den Wert des osmotischen Drucks einer Formulierung an. Die Messung der Osmolalität erfolgt anhand der Bestimmung der Gefrierpunktserniedrigung (mittels automatischen Halbmikroosmometer der Fa. KNAUER Wissenschaftliche Geräte GmbH, Deutschland) gemäß etablierter Prüfverfahren nach Ph. Eur 8.0, 2.2.35 und wird in mosmol/kg angegeben.

Es hat sich gezeigt, dass die Osmolalität und der damit verbundene osmotische Druck der Formulierung in der erfindungsmäßen Creme im Vergleich zum Referenzbeispiel deutlich erhöht ist.

4. Die erfindungsgemäße Creme aus Beispiel 3 sowie die Hautcreme aus dem Beispiel 4 wurden im Rahmen von Rezepturfreisetzungsversuchen ("in vitro release testing") analysiert. Die Freisetzung von Na-Dimethylglycinat aus den unterschiedlichen Rezepturen wurde mithilfe einer Phoenix DB-6 Diffusionszelle und anhand bereits etablierter Verfahren (siehe u.a. McKinney RD, Dunbar JR. In Vitro Release Tests of Ketoprofen from Pluronic Lecithin Organogel versus Lipoderm Using Immersion Cells and the Phoenix DB-6 Dry Heat Diffusion Tester. Int J Pharm Compd. 2021 May-Jun;25(3):241-245.) durchgeführt.

Für die Versuchsdurchführung wurde eine Polyethersulfonmembran (Porengröße 0,45 µm, Diameter 25 mm) in die Diffusionszelle eingespannt und eine 0.2 M Phosphatpuffer Lösung (pH 2.5) als Rezeptormedium bei 32 °C und 400 rpm Rührgeschwindigkeit verwendet. Es wurden 500 µL vom jeweiligen Produkt auf die Membran aufgebracht und nach 30, 60, 120 sowie 180 Minuten Proben aus dem Rezeptor entnommen und das entnommene Volumen jeweils mit frischen Rezeptormedium aufgefüllt. Der Gehalt an Na-Dimethylglycinat in den Proben wurde mittels HPLC-Methode ("high pressure liquid chromatography") bestimmt.

Es hat sich gezeigt, dass die Freisetzung von Na-Dimethylglycinat aus der erfindungsmäßen Creme mit einer Viskosität von 7500 mPas im Vergleich zum Beispiel mit einer höheren Viskosität von 32000 mPas deutlich schneller und besser erfolgt.

5. Die erfindungsgemäße Creme aus Beispiel 3 sowie die Hautcreme aus dem Beispiel 4 wurden im Rahmen von Penetrationsuntersuchungen an humanen Hautbiopsien analysiert. Die Penetration von Na-Dimethylglycinat aus den unterschiedlichen Rezepturen wurde mithilfe einer Franz-Diffusionszelle (Crown Glass Company INC, Somerville, New York, USA) durchgeführt. Vom Prinzip besteht die Franz-Zelle aus einer Donor- sowie Akzeptorkammer, die durch eine Hautbiopsie voneinander getrennt sind und stellt ein etabliertes System zur Untersuchung der Hautpenetration dar (siehe u.a. Trauer S, Patzelt A, Otberg N, Knorr F, Rozycki C, Balizs G, Büttemeyer R, Linscheid M, Liebsch M, Lademann J. Permeation of topically applied caffeine through human skin- -a comparison of in vivo and in vitro data. Br J Clin Pharmacol. 2009 Aug;68(2):181-6.). Die Apparatur wurde während der Durchführung auf 32°C temperiert und destilliertes Wasser als Rezeptormedium verwendet. Es wurden 20 mg Testprodukt in die Donorkammer auf Hautbiopsie (topische bzw. äußere Seite der Biopsie) aufgetragen und nach 1, 6 und 24 Stunden Hautstanzen entnommen. Für die Gehaltsbestimmungen von Na-Dimethylglycinat wurden die Hautstanzen nach etablierten Methoden aufgearbeitet und der Gehalt mittels HPLC-Methode ("high pressure liquid chromatography") in den einzelnen Hautschichten sowie im Rezeptormedium bestimmt.

Es hat sich gezeigt, dass die Penetration von Na-Dimethylglycinat in und durch die Haut aus der erfindungsmäßen Creme mit einer Viskosität von 7500 mPas im Vergleich zum Beispiel mit einer höheren Viskosität von 32000 mPas deutlich schneller und besser erfolgt.

6. Die erfindungsgemäße Creme aus Beispiel 5 und die Hautcreme aus dem Referenzbeispiel 4 wurden im Rahmen eines kontrollierten, verblindeten Halbseitentests auf dem Gesicht von 20 Frauen (Phototyp Fitzpatrick I-III, Alter: 20-64 Jahre) angewendet. Eine Menge von 80 µL (kontrollierte Volumenabmessung über Gilson Pipetman Microman E, M250E, 50-250 Mikroliter, 7-4305) wurde jeweils zeitgleich auf einer Gesichtshälfte der Probandinnen aufgetragen und gleichmäßig verteilt. Nach einer Minute (Einziehen der Creme in die Haut) wurden Parameter zur Wirksamkeit der Creme subjektiv durch die Bewertung der Probandinnen als auch objektiv durch die Bewertung einer geschulten und lang erfahrenen Kosmetikerin ermittelt. Zu den evaluierten Parametern zählten beispielsweise: Hautgefühl, Ebenmäßigkeit des Hautbildes, Hautfeuchtigkeit.

Es hat sich gezeigt, dass die Probandinnen selbst ein sehr viel gepflegteres und weicheres Hautgefühl erfahren haben durch die Auftragung der erfindungsmäßen Creme im Vergleich zum Referenzbeispiel. Gleichzeitig wurde das Hautbild objektiv als sehr viel gleichmäßiger bewertet nach Anwendung der Testcreme im Vergleich zum Referenzbeispiel.

## Patentansprüche

1. Topische Zusammensetzung enthaltend
a) Dimethylglycin und/oder ein Salz von Dimethylglycin,
b) mindestens eine Carbonsäure,
wobei das Gewichtsverhältnis von a) zu b) von 1:0,1 bis 1:10 reicht, zur Verwendung bei der Förderung von Wundheilung der Haut.

2. Zusammensetzung zur Verwendung gemäß Anspruch 1, wobei die Zusammensetzung 0,00001 bis 25,0 Gew.% Dimethylglycin und/oder ein Salz von Dimethylglycin enthält.

3. Zusammensetzung zur Verwendung gemäß Anspruch 1 und/oder Anspruch 2, wobei die Carbonsäure ausgewählt ist aus der Gruppe bestehend aus Adipinsäure, Ascorbinsäure, Milchsäure, Essigsäure, Propansäure, Glykolsäure, Brenztraubensäure, Oxalsäure, Äpfelsäure, Malonsäure, Bernsteinsäure, Maleinsäure, Fumarsäure, Weinsäure, Zitronensäure, Benzoesäure, Zimtsäure, Mandelsäure, Methansulfonsäure, Ethansulfonsäure, p-Toluolsulfonsäure, Salicylsäure, Azelainsäure, Zimtsäure, Ameisensäure, (Meth)Acrylsäure, Nicotinsäure sowie Mischungen daraus.

4. Zusammensetzung zur Verwendung gemäß einem der vorangehenden Ansprüche, wobei die Zusammensetzung Tenside in einer Menge von 2 bis 40 Gew.% enthält.

5. Zusammensetzung zur Verwendung gemäß einem der vorangehenden Ansprüche, wobei die Zusammensetzung mindestens einen weiteren Wirkstoff enthält, ausgewählt aus Koffein, Menthol, Biotin, Zink PCA, Niacinamid, Panthenol, Ectoin, Ubichinon-10, Taurin, Pantolacton, Echinacea, Carnitin, Tocopherylacetat und Kombinationen davon.

6. Zusammensetzung zur Verwendung gemäß einem der vorangehenden Ansprüche, wobei die Zusammensetzung mindestens ein Additiv enthält, ausgewählt aus Emulgatoren, Rückfettern, Konservierungsmitteln, Stabilisatoren, Duftstoffen, Antioxidantien, Rheologiemodifikatoren, Verdickungsmitteln, Pflegemitteln, Farbstoffen, Perlganzmitteln, Aufhellungsmitteln, Lösungsmitteln, Sonnenschutzmitteln und Kombinationen hiervon.
